# EUROPEAN PATENT SPECIFICATION

(11) **EP 2 271 318 B1**
(45) Date of publication and mention of the grant of the patent: **28.11.2012**
(21) Application number: 09730701.1
(22) Date of filing: 09.04.2009
(51) Int. Cl.: A61K 9/16, A61K 31/165

(54) **PHARMACEUTICAL DEPOT COMPRISING N-{5- T (CYCLOPROPYLAMINO) CARBONYL]-2-METHYLPHENYL}-3-FLUOR0-4- (PYRIDIN-2-YLMETHOXY) BENZAMIDE**
PHARMAZEUTISCHES DEPOT MIT N-{5- T (CYCLOPROPYLAMINO) CARBONYL]-2-METHYLPHENYL}-3-FLUOR-4- (PYRIDIN-2-YLMETHOXY)-BENZAMID
PRODUIT PHARMACEUTIQUE RETARD COMPRENANT DU N-{5-[(CYCLOPROPYLAMINO)CARBONYL]-2-MÉTHYLPHÉNYL}-3-FLUORO-4-(PYRIDIN-2-YLMÉTHOXY)BENZAMIDE

(30) Priority: 09.04.2008 US 43491
(43) Date of publication of application: 12.01.2011
(73) Proprietor: AstraZeneca AB, 151 85 Södertälje (SE)
(72) Inventor: BATEMAN, Nicola, Frances, Cheshire SK10 2NA (GB); MACFAUL, Philip, Alexander, Cheshire SK10 4TG (GB); NASH, Ian, Alun, Cheshire SK10 4TG (GB)
(74) Representative: Greaves, Carol Pauline
(86) International application number: PCT/GB2009/050353
(87) International publication number: WO 2009/125226

(56) References cited:
- WO-A-2005/061465
- US-A1- 2007 251 530

## Description

The present invention relates to a pharmaceutical depot comprising *N*-{5-[(cyclopropylamino)carbonyl]-2-methylphenyl}-3-fluoro-4-(pyridin-2-ylmethoxy)benzamide, or a pharmaceutically-acceptable salt thereof, and to uses of the pharmaceutical depot.

WO-A-2005/061465 discloses amide derivatives, including *N*-{5-[(cyclopropylamino)carbonyl]-2-methylphenyl}-3- fluoro-4-(pyridin-2-ylmethoxy)benzamide, and pharmaceutically-acceptable salts thereof, and teaches that the amide derivatives are inhibitors of the production of cytokines such as Tumour Necrosis Factor (hereinafter TNF), for example TNFα, and various members of the interleukin (hereinafter IL) family, for example IL-1, IL-6 and IL-8 (especially IL-1). In particular, and without wishing to imply that the amide derivatives disclosed in WO-A-2005/061465 possess pharmacological activity only by virtue of an effect on a single biological process, it is believed that the amide derivatives inhibit the effects of cytokines by virtue of inhibition of the enzyme p38 kinase. p38 kinase (otherwise known as cytokine suppressive binding protein, hereinafter CSBP) and reactivating kinase (hereinafter RK) is a member of the mitogen-activated protein (hereinafter MAP) kinase family of enzymes which is known to be activated by physiological stress such as that induced by ionising radiation, cytotoxic agents, and toxins, for example endotoxins such as bacterial lipopolysaccharide, and by a variety of agents such as the cytokines, for example TNFα and IL-1. It is known that p38 kinase phosphorylates certain intracellular proteins that are involved in the cascade of enzymatic steps which leads to the biosynthesis and excretion of cytokines such as TNFα and IL-1.

The amide derivatives disclosed in WO-A-2005/061465 therefore are believed to be useful in the treatment of diseases or medical conditions in which excessive production of cytokines occurs, for example excessive production of TNFα or IL-1. Such diseases and medical conditions include inflammatory and allergic diseases, such as inflammation of the joints (especially rheumatoid arthritis, osteoarthritis and gout). For the treatment of diseases and medical conditions such as inflammation of the joints, it would be desirable to administer the amide derivative directly to the site (such as the joint) requiring treatment, preferably so as to achieve a controlled- and/or sustained-release of the amide derivative at that site. Thus, there exists a need for a formulation or composition comprising *N*-{5-[(cyclopropylamino)carbonyl]-2-methylphenyl}-3-fluoro-4-(pyridin-2-ylmethoxy)benzamide, or a pharmaceutically-acceptable salt thereof, in a form suitable for such administration, for example for a pharmaceutical depot.

Although WO-A-2005/061465 suggests that the amide derivatives disclosed therein may be included in a pharmaceutical composition, for example in a form suitable for oral or topical use, for administration by inhalation or insufflation, or for parenteral administration, there is no disclosure in WO-A-2005/061465 of a pharmaceutical depot comprising an amide derivative as disclosed therein, let alone of such a pharmaceutical depot comprising *N*-{5-[(cyclopropylamino)carbonyl]-2-methylphenyl}-3-fluoro-4-(pyridin-2-ylmethoxy)benzamide, or a pharmaceutically-acceptable salt thereof.

According to the present invention, there is provided a pharmaceutical depot comprising (i) *N*-{5-[(cyclopropylamino)carbonyl]-2-methylphenyl}-3-fluoro-4-(pyridin-2-ylmethoxy)benzamide, or a pharmaceutically-acceptable salt thereof, as a pharmaceutical agent (PA) and (ii) a polymer which degrades to create an acidic microclimate, wherein the PA is released from the polymer upon polymer degradation.

In the pharmaceutical depot of the present invention, the pharmaceutical agent (hereinafter the PA) is *N*-{5-[(cyclopropylamino)carbonyl]-2-methylphenyl}-3-fluoro-4-(pyridin-2-ylmethoxy)benzamide, or a pharmaceutically-acceptable salt thereof. Thus, references herein to the PA include the compound *N*-{5-[(cyclopropylamino)carbonyl]-2-methylphenyl}-3-fluoro-4-(pyridin-2-ylmethoxy)benzamide *per se,* as well as pharmaceutically-acceptable salts thereof.

As the skilled person would appreciate, a pharmaceutical depot is a composition that releases a PA, especially a pharmaceutically effective amount of a PA (herein *N*-{5-[(cyclopropylamino)carbonyl]-2-methylphenyl}-3-fluoro-4-(pyridin-2-ylmethoxy)benzamide or a pharmaceutically-acceptable salt thereof) over time, so as to provide for the controlled- and/or sustained-release administration of the PA comprised therein.

*N*-{5-[(cyclopropylamino)carbonyl]-2-methylphenyl}-3-fluoro-4-(pyridin-2-ylmethoxy)benzamide has the structure: 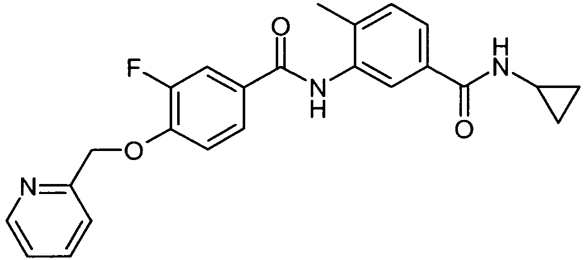 and is disclosed as example 5-y in WO-A-2005/061465.

Suitable pharmaceutically-acceptable salts of *N*-{5-[(cyclopropylamino)carbonyl]-2-methylphenyl}-3-fluoro-4-(pyridin-2-ylmethoxy)benzamide for including in the pharmaceutical depot of the present invention are based on reasonable medical judgement as being suitable for administration to a subject, for example a warm-blooded animal such as man, without undesirable pharmacological activities and without undue toxicity. Suitable pharmaceutically-acceptable salts include acid-addition salts, for example acid addition salts with an inorganic or organic acid such as hydrochloric, hydrobromic, sulfuric, phosphoric, trifluoroacetic, citric, maleic, tartaric, fumaric, hemifumaric, succinic, hemisuccinic, mandelic, methanesulfonic, dimethanesulfonic, ethane-1,2-sulfonic, benzenesulfonic, salicylic or 4-toluenesulfonic acid. A preferred acid addition salt is an acid addition salt with hydrochloric acid, i.e. to provide *N*-{5-[(cyclopropylamino)carbonyl]-2-methylphenyl}--3-fluoro-4-(pyridin-2-ylmethoxy)benzamide hydrochloride.

The *N*-{5-[(cyclopropylamino)carbonyl]-2-methylphenyl}-3-fluoro-4-(pyridin-2-ylmethoxy)benzamide, and pharmaceutically-acceptable salts thereof, may be synthesised from suitable starting materials using standard procedures of organic chemistry, for example as discussed in WO-A-2005/061465. For example, *N*-{5-[(cyclopropylamino)carbonyl]-2-methylphenyl}-3-fluoro-4-(pyridin-2-ylmethoxy)benzamide may be prepared by reaction of *N*-{5-[(cyclopropylamino)carbonyl]-2-methylphenyl}-3-fluoro-4-hydroxybenzamide with 2-chloromethyl-pyridine hydrochloride in the presence of a suitable base (such as potassium carbonate). Reaction of *N*-{5-[(cyclopropylamino)carbonyl]-2-methylphenyl}-3-fluoro-4-(pyridin-2-ylmethoxy)benzamide with hydrochloric acid provides the hydrochloride salt.

The pharmaceutical depot of the present invention enables the administration of the PA using a controlled- and/or sustained-release formulation so as to maintain a therapeutic level of the PA over an extended period of time. This is advantageous because it reduces the frequency of dosing and provides a convenient mode of administration of the PA, which is particularly desirable for the administration of the PA directly into the joint, i.e. by intra-articular administration. Controlled- and/or sustained-release formulations also can reduce the severity and frequency of any undesirable side effects associated with a particular PA. Improvements in the convenience of administration and reduced occurrence and severity of side effects in turn enhance patient compliance.

Many compounds that represent a PA are found to be unsuitable for including in pharmaceutical depots, primarily due to factors such as instability of the compounds in the formulations required for controlled- and/or sustained-release and/or for intra-articular administration. The present inventors have surprisingly found that the PA included in the pharmaceutical depot of the present invention is hydrolytically stable in the acidic microclimate created upon the degradation of the polymer included therein and therefore that the PA is suitable for including in the pharmaceutical depot. Furthermore, the present inventors have surprisingly found that the PA included in the pharmaceutical depot of the present invention can be provided at a sustained high local concentration of the PA at a site of administration, for example at a joint, to provide for the effective controlled- and/or sustained-release of the PA. In other words, the pharmaceutical depot is effective to slowly release the PA so as to achieve a long acting effect.

Advantageously, the PA may be included in the pharmaceutical depot of the present invention without any chemical modification being required prior to its inclusion therein.

As the skilled person would appreciate, a "pharmaceutical agent" (or PA) is an agent that causes a pharmacological effect in a subject to which it is administered, for example in a warm-blooded animal such as man, for example so as to treat or prevent a disease or medical condition. As discussed above, the PA in the pharmaceutical depot of the present invention is N-{5-[(cyclopropylamino)carbonyl]-2-methylphenyl}-3-fluoro-4-(pyridin-2-ylmethoxy)benzamide, or a pharmaceutically-acceptable salt thereof, which is believed to cause a pharmacological effect by means of the inhibition of the effects of cytokines (such as TNF, for example TNFα, and various members of the IL family, for example IL-1, IL-6 and IL-8) by virtue of inhibition of the enzyme p38 kinase.

The PA that is included in the pharmaceutical depot of the present invention is effective in the treatment of an inflammatory disease or condition, for example a condition caused by inflammation of a joint, such as osteoarthritis in which both acute and chronic synovial inflammation may occur. Osteoarthritis (also known as degenerative arthritis or degenerative joint disease) is the most common form of arthritis, with many sufferers worldwide and improved formulations for delivery of PAs for treatment of osteoarthritis are highly desirable.

As the skilled person will appreciate, the PA is present in the pharmaceutical depot of the present invention in a therapeutically effective amount. A "therapeutically effective amount" is any amount of the PA (for example as contained in the pharmaceutical depot) which, when administered to a subject suffering from a disease or medical condition against which the PA is effective, causes reduction, remission, or regression of the disease or medical condition.

The therapeutically effective amount of the PA that is included in the pharmaceutical depot will necessarily vary depending upon the nature and severity of the disorder to be treated and the particular patient treated, according to well known principles of medicine. Additionally, the therapeutically effective amount of the PA that is included in the pharmaceutical depot will necessarily vary according to the desired controlled-and/or sustained-release profile, for example depending on the period of time over which release of the PA is required and the concentration of PA desired over that time.

In addition to the PA, the pharmaceutical depot of the present invention comprises a polymer which degrades to create an acidic microclimate, for example a polymer which degrades in the presence of water to create an acidic microclimate. By this, we mean a polymer that degrades or breaks down chemically to provide an acidic pH in a small, localised area (such as a joint) to which the pharmaceutical depot is administered. Preferably, the acidic pH is essentially uniform in the localised area and differs from the surrounding area, which may be at a physiological pH (typically of about pH 7.4). The acidic pH is typically a pH of less than about 7.4, for example a pH in the range of from about I to about 7, such as from about 3 to about 7; conveniently from about 1 to less than 7 or from about 3 to less than 7.

Typically, the PA is dispersed or encapsulated in the polymer, such that the PA is continually released from the polymer as the polymer degrades over time to create the acidic microclimate. The PA that is included in the pharmaceutical depot of the present invention has been found to be hydrolytically stable in the acidic microclimate that is created by the degradation of the polymer. The release of the PA from the polymer provides for the controlled- and/or sustained-release of the PA from the pharmaceutical depot into a subject, for example a warm-blooded animal such as man, to which the pharmaceutical depot is administered. Preferably, a high local concentration (i.e. in the area to which the pharmaceutical depot is administered, such as a joint) to elicit the desired therapeutic effect and a low systemic concentration to mitigate against any undesired systemic toxicity of the PA is achieved upon polymer degradation and release of the PA. Thus, the pharmaceutical depot delivers the PA to the subject at concentrations effective for treatment of the particular disease or medical condition over a sustained period of time.

Any suitable polymer may be used in the pharmaceutical depot of the present invention, provided that the polymer degrades to create an acidic microclimate, i.e. upon administration to a subject, for example to a warm-blooded animal such as man, and is biodegradable and biocompatible.

As the skilled person would appreciate, by the term "biocompatible" we mean a material that is compatible with living tissue or a living system by not being toxic, injurious, or physiologically reactive and not causing immunological rejection.

By the term "biodegradable" we mean a material that is degraded in a biological environment.

For example, a polymer may be "biodegradable" such that the entire polymer biodegrades and does not need to be removed after use, i.e. once all of the PA has been released. Such polymers may comprise hydrolysable and enzymatically cleavable ester linkages that break down under biological conditions (for example in the presence of water and biological enzymes found in tissues of warm-blooded animals such as humans) to produce non-toxic, biocompatible and/or biodegradable products. Alternatively, a polymer may be "biodegradable" by virtue of having a finite half-life in a biological environment. For example the polymer may have a half-life of from 1 to 12 months, such as from 1 to 6 months.

Typically, the polymer includes at least one acidic functional group or at least one functional group that may react to produce an acidic functional group, i.e. which acidic functional group is a group that is capable of donating a proton to a basic functional group such as an amine. Examples of suitable acidic functional groups include carboxylic acid groups (i.e. -CO₂H) and sulfonic acid groups (i.e. -S(O)₂0H). Examples of suitable functional groups that may react to produce an acidic functional group include esters (i.e. RC(O)OR, where R may represent alkyl or aryl), which esters may react with water to produce a corresponding carboxylic acid group and an alcohol.
Preferably, the polymer is selected so as to degrade and release the PA over a period of from 30 to 90 days. For example, the polymer may degrade and release the PA over a period of 30, 60 or 90 days. For example, the polymer may degrade and release the PA over a period of 120, 150 or 180 days.

Suitable polymers include a polyester of a hydroxyfatty acid and derivatives thereof (for example polylactic acid, polyglycolic acid, polycitric acid, polymalic acid, poly-β-hydroxybutyric acid, ε-capro-lactone ring opening polymer, lactic acid-glycolic acid copolymer, 2-hydroxybutyric acid-glycolic acid copolymer, polylactic acid-polyethyleneglycol copolymer or polyglycolic acid-polyethyleneglycol copolymer), a polymer of an alkyl α-cyanoacrylate (for example poly(butyl 2-cyanoacrylate)), a polyalkylene oxalate (for example polytrimethylene oxalate or polytetramethylene oxalate), a polyortho ester, a polycarbonate (for example polyethylene carbonate or polyethylenepropylene carbonate), a polyortho-carbonate, a polyamino acid (for example poly-γ-L-alanine, poly-γ-benzyl-L-glutamic acid or poly-γ-methyl-L-glutamic acid), a hyaluronic acid ester, and the like, and one or more of these polymers can be used.

If the polymers are copolymers they may be any of random, block and graft copolymers. When the above α-hydroxycarboxylic acids, hydroxydicarboxylic acids and hydroxytricarboxylic acids have optical activity in their molecules, any one of D-isomers, L-isomers and DL-isomers may be used. Among others, α-hydroxycarboxylic acid polymer (preferably lactic acid-glycolic acid polymer), its ester, poly-α-cyanoacrylic acid esters, etc. are preferred, and lactic acid-glycolic acid copolymer (also referred to as poly(lactide-co-glycolide) or poly(lactic-co-glycolic acid), and hereinafter referred to as PLGA) are most preferred. Thus, in one aspect the polymer is PLGA. As used herein, the term PLGA includes polymers of lactic acid (also referred to as polylactide, poly(lactic acid), or PLA).

Suitable PLGA polymers may have a molar ratio of lactic acid:glycolic acid in the range of 100:0 to 50:50, conveniently in the range of 95:5 to 50:50. For example, the PLGA polymer may have a molar ratio of lactic acid:glycolic acid of 95:5 or of 50:50.

Suitable PLGA polymers may have a block length in the range of from 1 to 5, preferably of from 2 to 4.

Suitable PLGA polymers may have a weight-average molecular weight of from about 3,000 to about 50,000, preferably of about 4,000 to about 40,000, and more preferably of about 5,000 to about 30,000 Daltons. The degree of dispersion (weight-average molecular weight/number-average molecular weight, hereinafter referred to as polydispersity) may range from about 1.2 to about 4.0, preferably from about 1.3 to about 3.5.

As the skilled person would appreciate, the weight-average molecular weight, number-average molecular weight and polydispersity may be determined by any suitable method or means, for example by size exclusion chromatography (SEC) with narrow polydispersity polystyrene reference substances with peak molecular weights of 1,000,000, 130,000, 50,000, 20,000, 10,000, 5,000, 2,000, and 580 respectively. The determination may be carried out using a SEC column Mixed Bed D 5µm (manufactured by Polymer Laboratories Ltd., UK) and using 5% methanol in tetrahydrofuran as the mobile phase.

The PLGA may be prepared by any conventional method, or may be commercially available. For example, PLGA can be produced by ring-opening polymerisation with a suitable catalyst from cyclic lactide, glycolide, etc. (see Encyclopedic Handbook of Biomaterials and Bioengineering Part A: Materials, Volume 2, Marcel Dekker, Inc. (1995); EP-0058481B2; Effects of polymerization variables on PLGA properties: molecular weight, composition and chain structure and Dorta et al, Int. J. Pharm., 100, pp 9-14 (1993)).

It is believed that PLGA is biodegradable by means of the degradation of the entire solid polymer composition, due to the break down of hydrolysable and enzymatically cleavable ester linkages under biological conditions (for example in the presence of water and biological enzymes found in tissues of warm-blooded animals such as humans) to form lactic acid and glycolic acid. Both lactic acid and glycolic acid are water-soluble, non-toxic products of normal metabolism, which may further biodegrade to form carbon dioxide and water. In other words, PLGA is believed to degrade by means of hydrolysis of its ester groups in the presence of water, for example in the body of a warm-blooded animal such as man, to produce lactic acid and glycolic acid and create the acidic microclimate. Lactic and glycolic acid are by-products of various metabolic pathways in the body of a warm-blooded animal such as man under normal physiological conditions and therefore are well tolerated and produce minimal systemic toxicity.

The polymer is provided in any suitable form in which the PA may be dispersed or encapsulated therein prior to the degradation of the polymer. For example, the pharmaceutical depot may comprise the polymer in the form of microparticles or nanoparticles, or in a liquid form, with the PA dispersed or encapsulated therein.

Suitable microparticles typically have an average particle size in the range of 0.1 to 1000µm, preferably 1 to 750µm and more preferably 10 to 500µm.

Suitable nanoparticles typically have an average particle size in the range of 1 to 2000nm, preferably 10 to 1000nm, and more preferably 50 to 500nm.

In particular, the microparticles are substantially spherical in shape (ie. are microspheres).

When the polymer is in the form of microparticles, the microparticles may be prepared using any appropriate method, such as by a solvent evaporation or solvent extraction method. For example, in the solvent evaporation method, the PA and the polymer may be dissolved in a suitable volatile organic solvent (for example a ketone such as acetone, a halogenated hydrocarbon such as chloroform or methylene chloride, a halogenated aromatic hydrocarbon, a cyclic ether such as dioxane, an ester such as ethyl acetate, a nitrile such as acetonitrile, or an alcohol such as ethanol) and dispersed in an aqueous phase containing a suitable emulsion stabiliser (for example polyvinyl alcohol, PVA). The organic solvent is then evaporated to provide microparticles with the PA encapsulated therein. In the solvent extraction method, the PA and polymer may be dissolved in a polar solvent (such as acetonitrile, dichloromethane, methanol, ethyl acetate or methyl formate) and then dispersed in an aqueous phase (such as a water/PVA solution). An emulsion is produced to provide microparticles with the PA encapsulated therein. Spray drying is an alternative manufacturing technique for preparing the microparticles.

In one aspect, the pharmaceutical depot may comprise the polymer (such as PLGA as described above) in the form of microparticles with the PA encapsulated therein. For example, the pharmaceutical depot may comprise a PLGA polymer having a lactide:glycolide molar ratio of 50:50 in the form of microparticles with the PA encapsulated therein. Such a pharmaceutical depot may be suitable for controlled- and/or sustained-release of the PA over a period of about 30 days. Further, as an example, the pharmaceutical depot may comprise a PLGA polymer having a lactide:glycolide molar ratio of 95:5 in the form of microparticles with the PA encapsulated therein. Such a pharmaceutical depot may be suitable for controlled- and/or sustained-release of the PA over a period of from about 60 to 90 days. Such a pharmaceutical depot may also be suitable for controlled- and/or sustained-release of the PA over a period of up to 120, up to 150, or up to 180 days.

The pharmaceutical depot may comprise the PA and the polymer in any suitable amounts. For example, the pharmaceutical depot may comprise from 1 to 30% by weight of the PA and from 70 to 99% by weight of the polymer.

For example, when the pharmaceutical depot of the present invention comprises PLGA microparticles, the PLGA may be present in an amount ranging from about 70% to about 99% by weight of the microparticles. This amount of PLGA may be used when about 1% to about 30% by weight of the PA is loaded into the microparticles. Also, this amount of the polymer is calculated for the microparticles comprising the PA and the PLGA , but not other pharmaceutical excipients, for example used for suspending the microparticles before lyophilisation. The PLGA may be used in an amount of from about 88% to about 90% by weight of the microparticles, when about 10% to about 12% by weight of the PA is loaded in the microparticles. The proportion of the polymer typically depends on the strength of pharmacological activity of the PA used and the rate and duration of release of the PA.

The pharmaceutical depot may further comprise a suitable pharmaceutically-acceptable diluent or carrier, which should be water miscible. Suitable diluents or carriers include, for example, suitable porosity-modifying agents (such as sodium chloride) that rapidly dissolve leaving pores and/or suitable plasticisers to modify the rate of diffusion and/or reduce porosity (see, for example, Burgess, D. J., Hickey, A. J., Drugs and the Pharmaceutical Sciences (149) pp 305-353).

The diluent or carrier may be included in the pharmaceutical depot in any suitable amount. For example, the diluent or carrier may be included in an amount of from 0 to 50% by weight of the total composition. Preferably, the pharmaceutical depot does not contain an additional diluent or carrier.

The pharmaceutical depot typically is provided for local delivery at a desired site of treatment, such as at a joint.

The pharmaceutical depot may be formulated for administration by injection, such as by intra-articular injection. Thus, in particular, the pharmaceutical depot may be provided in an injectable form (i.e. as an injectable pharmaceutical depot). By "injectable" we mean that the pharmaceutical depot can be drawn into a syringe and injected into a subject, for example a warm-blooded animal such as man, without causing adverse effects due to the presence of solid material in the depot. For example, the pharmaceutical depot may be injectable into a joint, such as an inflamed joint. In other words, there is provided a pharmaceutical depot for intra-articular injection. Suitable joints include knee, hip, shoulder, ankle, elbow, wrist, toe, finger and spinal facet joints. The pharmaceutical depot remains in the joint after injection thereto and achieves a local delivery of the PA in a controlled and sustained manner, preferably over a period of time ranging from 30 to 90 days. Pharmaceutical depots that achieve a local delivery of the PA in a controlled and sustained manner over a period of up to 90 days are advantageous because this minimises the number of local injections required to be made to a joint, which enables the depots to meet current recommendations for intra-articular therapy which advise not to exceed three to four small (about 2 ml) local injections into a joint per year due to possible adverse effects.

The pharmaceutical depot may be formulated for injection into the intra-articular space of an affected joint, for example into the synovial fluid-containing portion of an affected joint, such as at an osteoarthritis site. As skilled person would appreciate the synovial fluid is contained within a central joint space defined by opposing bones of the joints. The present inventors have found that upon injection of the pharmaceutical depot into the synovial fluid, the PA is released and substantially enters the surrounding tissue with only minor amounts entering the blood stream, i.e. to achieve a high local concentration of PA in the area to which the pharmaceutical depot is administered (such as a joint) and a low systemic concentration. Additionally, the pharmaceutical depot provides an acceptable "burst" (i.e. release of PA) on the first day following administration, which is advantageous in use and is unexpected in view of the teaching of the prior art, such as in US-6,217,911, which teaches that little or no burst release is preferred. The efficient release profile provided by the pharmaceutical depot of the present invention would not have been predicted from the prior art and aids in the effectiveness of the pharmaceutical depot.

Preferably, the pharmaceutical depot provides a sustained high local concentration of the PA in an articular joint upon administration by injection thereto, such as above 100 nanomolar.

Injectable pharmaceutical depots may comprise a suspension or dispersion of the PA and polymer combination in a pharmaceutically-acceptable diluent or carrier, which should be water miscible. Suitable diluents or carriers include aqueous diluents or carriers such as an isotonic aqueous solution of a viscosity improver (such as sodium carboxymethylcellulose), a surfactant (such as polysorbate 80) and/or a tonicity adjuster (such as sodium chloride). Injectable pharmaceutical depots may comprise further active agents, such as a local anaesthetic.

The pharmaceutical depot of the present invention may be formulated for human medicine or veterinary use. For example, there may be provided a pharmaceutical depot formulated for intra-articular injection for human medicine or veterinary use.

The present invention further provides a pharmaceutical depot as defined herein for use in inhibiting the effects of cytokines, for example by virtue of the inhibition of the enzyme p38 kinase, in a subject.

According to another aspect of the present invention, there is provided the use of a pharmaceutical depot as defined herein for inhibiting the effects of cytokines, for example by virtue of the inhibition of the enzyme p38 kinase, in a subject.

According to another aspect of the present invention, there is provided the use of a pharmaceutical depot as defined herein in the manufacture of a medicament for use in inhibiting the effects of cytokines, for example by virtue of the inhibition of the enzyme p38 kinase, in a subject.

According to another aspect of the present invention, there is provided a method for inhibiting the effects of cytokines, for example by virtue of the inhibition of the enzyme p38 kinase, in a subject in need thereof, which method comprises administering to said subject a pharmaceutical depot as defined herein.

The present invention further provides a pharmaceutical depot as defined herein for use in the prevention or treatment of an inflammatory disease, such as osteoarthritis, in a subject.

According to another aspect of the present invention, there is provided the use of a pharmaceutical depot as defined herein for the prevention or treatment of an inflammatory disease, such as osteoarthritis, in a subject.

According to another aspect of the present invention, there is provided the use of a pharmaceutical depot as defined herein in the manufacture of a medicament for use in the prevention or treatment of an inflammatory disease, such as osteoarthritis, in a subject.

According to another aspect of the present invention, there is provided a method for the prevention or treatment of an inflammatory disease, such as osteoarthritis, in a subject in need thereof, which method comprises administering to said subject a pharmaceutical depot as defined herein.

The "subject" to which the pharmaceutical depot of the invention is to be administered is an animal, especially a warm-blooded animal, such as a domestic animal or man, particularly man.

The invention will now be illustrated by the following non-limited examples.

### Example 1

A pharmaceutical depot was prepared that comprised PLGA microparticles encapsulating *N*-{5-[(cyclopropylamino)carbonyl]-2-methylphenyl}-3-fluoro-4-(pyridin-2-ylmethoxy)benzamide as the PA.

### (i) Microparticle Preparation

60 mg of *N*-{5-[(cyclopropylamino)carbonyl]-2-methylphenyl}-3-fluoro-4-(pyridin-2-ylmethoxy)benzamide and 340 mg of PLGA (molar lactide:glycolide ratio of 50:50 and a molecular weight of 19.5 KD) were dissolved in dichloromethane/Methanol 3:1 ratio (2 ml). This solution was then dispersed in an aqueous phase of 0.5% PVA w/v under high shear to form an emulsion. The high shear was created by using a static mixer with a high flow rate of aqueous phase e.g. 1000mls/min. The resulting emulsion was added to water (1250 ml) at 30°C and stirred at 500rpm (using a Heidolph RZR1 stirrer) for 1 hour. The resulting suspension was cooled in an ice bath and the microparticles allowed to sediment for 45 minutes. Approximately 90% (by volume) of the supernatant was removed, taking care not to disturb the sedimented microparticles. Water (1 L) was added and the process repeated. Approximately 95% (by volume) of the supernatant was removed and the microparticles transferred to a glass test tube. The wash/sedimentation cycle was repeated a further 2 times and the microparticles were transferred to a freeze dry vial with the minimum volume of water. The vial was flash frozen in liquid nitrogen and the microparticles were freeze-dried for 48 hours.

### (ii) In Vitro Release Protocol

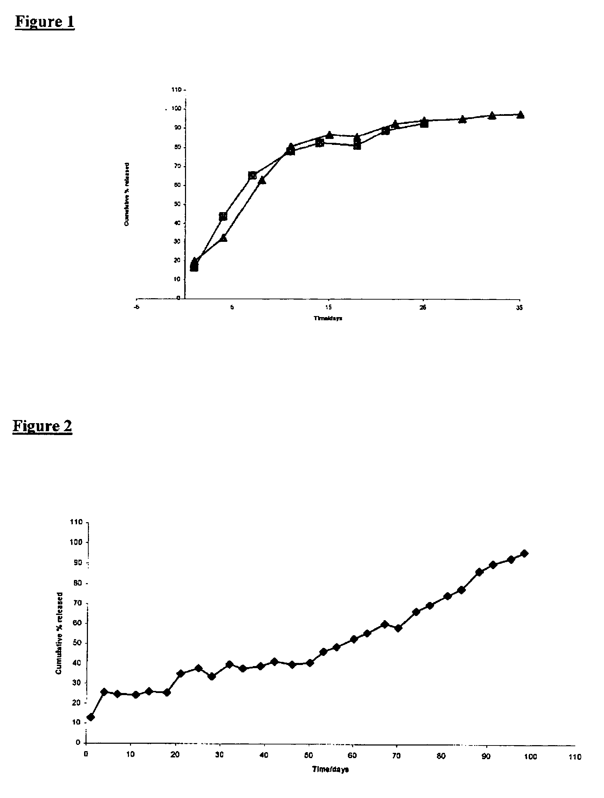

0.8 mg of microparticles containing *N*-{5-[(cyclopropylamino)carbonyl]-2-methylphenyl]-3-fluoro-4-(pyridin-2-ylmethoxy)benzamide in 50:50 PLGA were suspended in PBS containing 0.1% w.v Tween 80 (20 ml). The resultant slurry was kept static at 37°C and samples were taken at 24 hours by removal of media (1 ml) followed by addition on media (1 ml) to ensure the volume of media within the experiment remained constant. Samples were taken at regular intervals (see Figure 1) until the depot was no longer releasing *N*-{5-[(cyclopropylamino)carbonyl]-2-methylphenyl}-3-fluoro-4-(pyridin-2-ylmethoxy)benzamide and analysed by HPLC. The results are shown in Table 1 below.

**Table 1**

| **Polymer lactide:glycolide molar ratio / MW (KD)** | **PA load (% by weight)** | **Encapsulation Efficiency (%)** | **In vitro Burst (%)** | **In vitro Release (%)** |
|---|---|---|---|---|
| 50:50 | 13.33 | 88.87 | 16.33 | Day 14 - 82.3 |
| 19.5 | | | | Day 25 - 92.38 |
| 50:50 | 13.60 | 90.67 | 18.35 | Day 15 - 79.52 |
| 19.5 | | | | Day 25 - 86.23 |

The microparticles with 50:50 PLGA provided high encapsulation efficiencies, producing *N*-{5-[(cyclopropylamino)carbonyl]-2-methylphenyl}-3-fluoro-4-(pyridin-2-ylmethoxy)benzamide loads of about 13%. The *in vitro* release profile data is shown in Figure 1. The *in vitro* release studies show that the *N*-{5-[(cyclopropylamino)carbonyl]-2-methylphenyl}-3-fluoro-4-(pyridin-2-ylmethoxy)benzamide in 50:50 PLGA microparticles had an acceptable burst on day one and released over 1 month *in vitro.* The two batches produced using 50:50 PLGA (Table 1) showed good reproducibility.

### Example 2

A pharmaceutical depot was prepared that comprised PLGA microparticles encapsulating *N*-{5-[(cyclopropylamino)carbonyl]-2-methylphenyl}-3-fluoro-4-(pyridin-2-ylmethoxy)benzamide as the PA.

### (i) Microparticle Preparation

60 mg of *N*-{5-[(cyclopropylamino)carbonyl]-2-methylphenyl}-3-fluoro-4-(pyridin-2-ylmethoxy)benzamide and 340 mg of PLGA (molar lactide:glycolide ratio of 95:5 and a molecular weight of 23 KD) were dissolved in dichloromethane/Methanol 3:1 ratio (2 ml). This solution was then dispersed in an aqueous phase of 0.5% PVA w/v under high shear to form an emulsion. The high shear was created by using a static mixer with a high flow rate of aqueous phase e.g. 1000mls/min. The resulting emulsion was added to water (1250 ml) at 30°C and stirred at 500 rpm (using a Heidolph RZR1 stirrer) for 1 hour. The resulting suspension was cooled in an ice bath and the microparticles allowed to sediment for 45 minutes. Approximately 90% by volume of the supernatant was removed taking care not to disturb the sedimented microparticles. Water (1L) was added and the process repeated. Approximately 95% by volume (of the supernatant was removed and the microparticles transferred to a glass test tube. The wash/sedimentation cycle was repeated a further 2 times and the microparticles were transferred to a freeze dry vial with the minimum volume of water. The vial was flash frozen in liquid nitrogen and the microparticles were freeze-dried for 48 hours.

### (ii) In Vitro Release Protocol

0.8 mg of microparticles containing *N*-{5-[(cyclopropylamino)carbonyl]-2-methylphenyl}-3-fluoro-4-(pyridin-2-ylmethoxy)benzamide in 95:5 PLGA were suspended in PBS containing 0.1% w.v Tween 80 (20 ml). The resulting slurry was kept static at 37°C and samples were taken at 24 hours by removal of media (1 ml) followed by addition on media (1 ml) to ensure the volume of media within the experiment remained constant. Samples were taken at regular intervals (see Figure 2) until the depot was no longer releasing *N*-{5-[(cyclopropylamino)carbonyl]-2-methylphenyl}-3- fluoro-4-(pyridin-2-ylmethoxy)benzamide and analysed by HPLC. The results are shown in Table 2 below.

**Table 2**

| **Polymer lactide:glycolide molar ratio / MW (KD)** | **PA load (% by weight)** | **Encapsulation Efficiency (%)** | **In vitro Burst (%)** | **In vitro Release (%)** |
|---|---|---|---|---|
| 95:5 | 12.95 | 86.33 | 12.78 | Day 46 - 39.84 |
| 23 | | | | Day 91 - 90.02 |

The microparticles with *N*-{5-[(cyclopropylamino)carbonyl]-2-methylphenyl]-3-fluoro-4-(pyridin-2-ylmethoxy)benzamide provided high encapsulation efficiencies, producing PA loads of about 13%. The full *in vitro* release profile data is shown in Figure 2. The *in vitro* release studies show that the *N*-{5-[(cyclopropylamino)carbonyl]-2-methylphenyl}-3-fluoro-4-(pyridin-2-ylmethoxy)benzamide in 95:5 PLGA microparticles had an acceptable burst on day one and released over 3 months *in vitro.*

### Example 3

The release characteristics of *N*-{5-[(cyclopropylamino)carbonyl]-2-methylphenyl}-3-fluoro-4-(pyridin-2-ylmethoxy)benzamide in 50:50 PLGA microparticles *in vivo* in the rat were investigated.

Unformulated *N*-{5-[(cyclopropylamino)carbonyl]-2-methylphenyl]-3-fluoro-4-(pyridin-2-ylmethoxy)benzamide was intra-articularly injected (15ng in 5 µl injection in PBS) to rats and synovial fluid concentrations were determined at 15, 30 and 60 minutes post dose. The synovial fluid from the rat knee joint was sampled using a knee wash methodology. The knee was exposed and a transverse cut made to the patellar tendon proximal to the tibia. The knee cavity was opened up by dissection, and the knee lavaged between the tibial and femoral condyles with 3x25µl PBS using an eppendorf pipette. Pharmacokinetic parameters of the PA in synovial fluid calculated from this experiment are shown in Table 3 below:

**Table 3**

| Parameter | Value | Units |
|---|---|---|
| Clearance (Cl) | 61 | µl/hour |
| Volume of distribution (Vdss) | 9 | µl |
| Half-life (t_{1/2}) | 0.2 | hour |

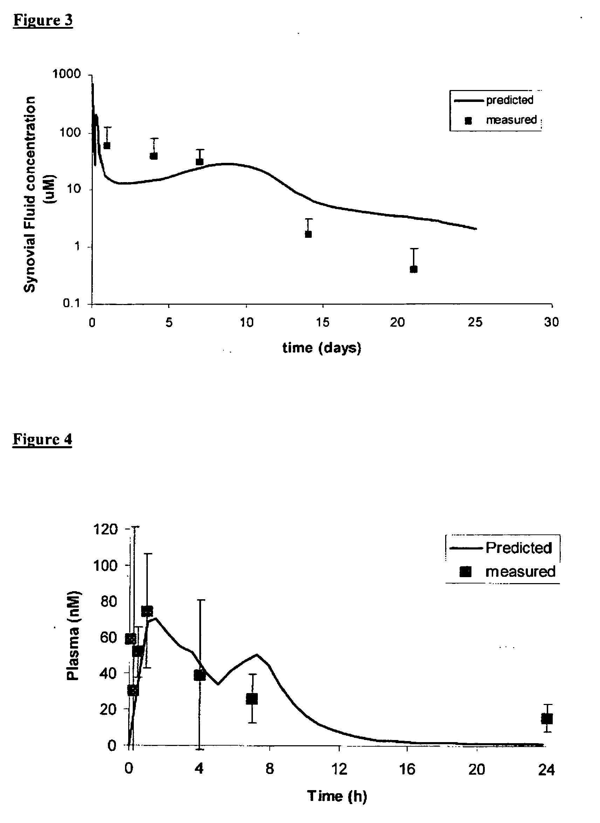

*N-*{5-[(cyclopropylamino)carbonyl]-2-methylphenyl}-3-fluoro-4-(pyridin-2-ylmethoxy)benzamide as the PA in 50:50 PLGA microparticles (as prepared in Example 1) was then dosed intra-articularly (200 µg in 30µl) to rats and synovial fluid concentrations were determined on days 1, 4, 7, 14 and 21 post dose. Data obtained in this study is graphically shown in Figure 4, together with a simulation of the expected synovial fluid concentrations based on the *in-vitro* release characteristics of this formulation (see Example 1) and the calculated clearance of released drug out of the synovial fluid (Cl = 61 µl/hour).

This data clearly demonstrates that the *N*-{5-[(cyclopropylamino)carbonyl]-2-methylphenyl}-3-fluoro-4-(pyridin-2-ylmethoxy)benzamide as the PA in 50:50 PLGA microparticles when injected intra-articularly to rats can sustain release in the synovial fluid for 21 days. In addition, the good agreement between the predicted concentrations and the measured concentrations, suggests that the *in vitro* release assay is a good predictor of the *in vivo* behaviour for this formulation.

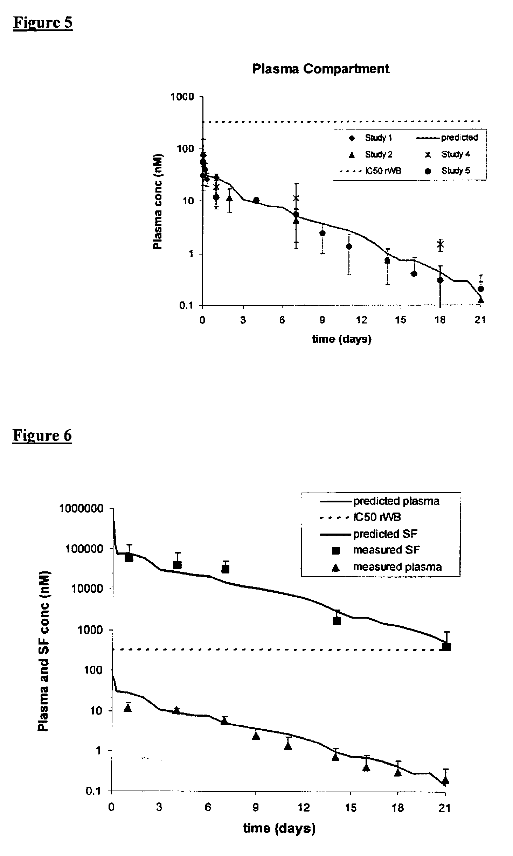

*N*-{5-[(cyclopropylamino)carbonyl]-2-methylphenyl}-3-fluoro-4-(pyridin-2-ylmethoxy)benzamide as the PA in 50:50 PLGA microparticles (as in Example 1) was then dosed intra-articularly (200 µg) to rats and plasma concentrations were determined up to 24 hours and 21 days post dose. The data obtained is graphically shown in Figure 4 and Figure 5 respectively, together with a simulation of the expected plasma concentrations based on the *in vitro* burst release characteristics, the calculated clearance of released drug out of the synovial fluid (Cl = 61 µl/hour) and the systemic pharmacokinetic parameters of this compound in the rat (Cl = 14 ml/min/kg, Vdss = 1.71/kg)

As shown in Figure 4 and 5, the plasma concentrations of the PA were in the nanomolar range (compared to the micromolar range for synovial fluid, as shown in Figure 3) confirming the concept that intra-articular delivery by means of the pharmaceutical depot of the present invention can effectively buffer systemic exposure even during peak efflux of the PA from depot formulations. A summary of this data is presented on the same scale in Figure 6 (wherein "predicted SF" is the upper line and "predicted plasma" is the lower line) . The microparticles injected intra-articularly showed only a small amount of PA loss due to burst effects leading to low plasma concentrations and therefore minimizing the risk for toxicity.

In summary, the pharmaceutical depot comprising PA in PLGA microparticles when injected intra-articularly (200 µg) to rats can sustain release in the synovial fluid for up to 21 days and leads to very low plasma concentrations shortly after dosing due to a reduced burst effect. Moreover, the *in vitro* release assay is a good predictor of the *in vivo* behaviour for 50:50 PLGA microparticles.

### Example 4

The release characteristics of *N*-{5-[(cyclopropylamino)carbonyl]-2-methylphenyl }-3-fluoro-4-(pyridin-2-ylmethoxy)benzamide in 50:50 PLGA microparticles *in vivo* in the rat were investigated.

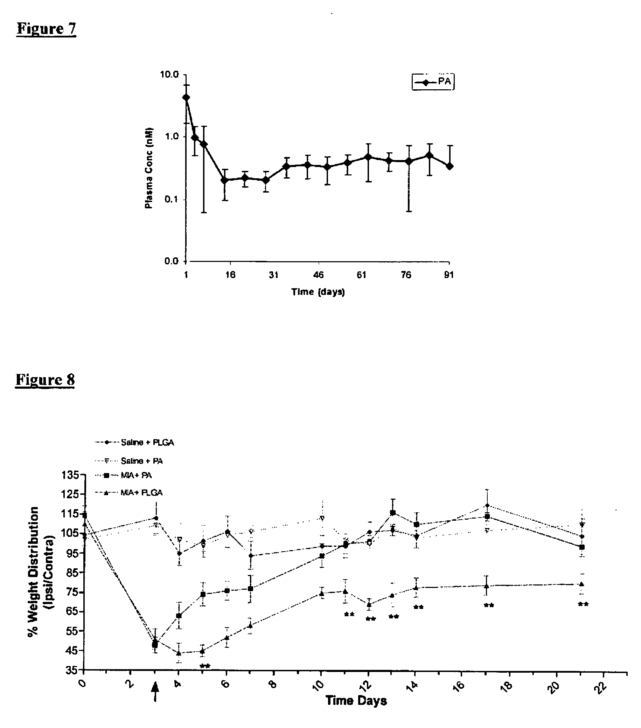

*N*-{5-[(cyclopropylamino)carbonyl]-2-methylphenyl}-3-fluoro-4-(pyridin-2-ylmethoxy)benzamide as the PA in 50:50 PLGA microparticles (as in Example 2) was then dosed intra-articularly (200 µg) to rats and plasma concentrations were determined up to 91 days post dose. The data obtained is graphically shown in Figure 7 (which shows the *in vivo* release profile of the PA in 95:5 PLGA mciroparticles in rats).

In summary, the pharmaceutical depot comprising PA in PLGA microparticles when injected intra-articularly (200 µg) to rats demonstrates a release profile within plasma for 91 days giving very low plasma concentrations shortly after dosing due to a reduced burst effect.

### Example 5

The sustained efficacy of *N*-{5-[(cyclopropylamino)carbonyl]-2-methylphenyl]-3-fluoro-4-(pyridin-2-ylmethoxy)benzamide as the PA in a pharmaceutical depot was investigated.

All studies were carried out in a rat mono-iodoacetate (MIA) model of joint pain as a screen for analgesia of pain driven by joint inflammation and destruction (see Ivanavicius et al., 2007 Pain 128 p272). The MIA model induces an early synovitis (day 3) followed by progressive loss of articular cartilage, and subchondral bone pathology by day 14.

*N-*{5-[(cyclopropylamino)carbonyl]-2-methylphenyl}-3-fluoro-4-(pyridin-2-ylmethoxy)benzamide was formulated into PLGA microspheres (50:50 PLGA as in Example 1) and tested in the MIA model. Rats were injected intra-articularly with MIA on day 0. Three days post MIA (to allow disease to progress) animals were injected intra-articularly with formulated *N*-{5-[(cyclopropylamino)carbonyl]-2-methylphenyl}-3-fluoro-4-(pyridin-2-ylmethoxy)benzamide in 50:50 PLGA (200 µg/30 µl) or with microsphere formulation (30 µl). The data are shown in Figure 3, which data clearly show that there is an immediate and sustained efficacy following the injection of the formulated *N*-{5-[(cyclopropylamino)carbonyl]-2-methylphenyl}-3-fluoro-4-(pyridin-2-ylmethoxy)benzamide. This normalisation of weight bearing asymmetry is statistically significant 48 hours post dose and from day 6 post dose until the termination of the study (18 days post dose) is shown graphically in Figure 8.

This demonstrates a sustained efficacy is achievable using formulated *N*-{5-[(cyclopropylamino)carbonyl]-2-methylphenyl}-3-fluoro-4-(pyridin-2-ylmethoxy)benzamide in PLGA microspheres. There was complete reversal of weight bearing asymmetry.

### Example 6

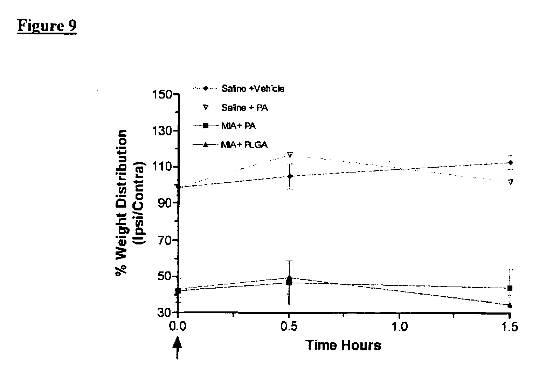

A comparable study to Example 5 was carried out to assess the effects of unformulated N-{5-[(cyclopropylamino)carbonyl]-2-methylphenyl}-3-fluoro-4-(pyridin-2-ylmethoxy)benzamide. A dose of 29 µg/ml (69 µM) of N-{5-[(cyclopropylamino)carbonyl]-2-methylphenyl}-3-fluoro-4-(pyridin-2-ylmethoxy)benzamide was delivered in a 5 µl injection volume to give a Cmin concentration of 1 µM at 1.5 hours. The dosing was carried out 3 days post MIA at the same time point that formulated N-{5-[(cyclopropylamino)carbonyl]-2-methylphenyl}-3-fluoro-4-(pyridin-2-ylmethoxy)benzamide as the PA were dosed. Data is shown in Figure 9, which clearly show no efficacy of unformulated N-{5-[(cyclopropylamino)carbonyl]-2-methylphenyl}-3-fluoro-4-(pyridin-2-ylmethoxy)benzamide in the MIA model at day 3 indicating an absolute requirement to depot formulated N-{5-[(cyclopropylamino)carbonyl]-2-methylphenyl}-3-fluoro-4-(pyridin-2-ylmethoxy)benzamide in the joint for sustained periods to realise a pharmacodynamic effect.

## Claims

1. A pharmaceutical depot comprising (i) *N*-{5-[(cyclopropylamino)carbonyl]-2-methylphenyl}-3-fluoro-4-(pyridin-2-ylmethoxy)benzamide, or a pharmaceutically-acceptable salt thereof, as a pharmaceutical agent (PA) and (ii) a polymer which degrades to create an acidic microclimate, wherein the PA is released from the polymer upon polymer degradation.

2. The pharmaceutical depot according to claim 1, wherein the polymer is selected from a polyester of a hydroxyfatty acid and derivatives thereof, a polymer of an alkyl α-cyanoacrylate, a polyalkylene oxalate, a polyortho ester, a polycarbonate, a polyortho-carbonate, a polyamino acid, a hyaluronic acid ester, and mixtures thereof.

3. The pharmaceutical depot according to claim 2, wherein the polymer is a lactic acid-glycolic acid copolymer.

4. The pharmaceutical depot according to claim 3, wherein the lactic acid-glycolic acid copolymer has a molar ratio of lactic acid:glycolic acid in the range of 100:0 to 50:50.

5. The pharmaceutical depot according to claim 4, wherein the lactic acid-glycolic acid copolymer has a molar ratio of lactic acid:glycolic acid of 95:5.

6. The pharmaceutical depot according to claim 4, wherein the lactic acid-glycolic acid copolymer has a molar ratio of lactic acid:glycolic acid of 50:50.

7. The pharmaceutical depot according to any one of claims 1 to 6, wherein the pharmaceutical depot is formulated for controlled- and/or sustained-release of the PA over a period of from 30 to 90 days.

8. The pharmaceutical depot according to claim 7, wherein the pharmaceutical depot is formulated for controlled- and/or sustained-release of the PA over a period of 30 days.

9. The pharmaceutical depot according to claim 7, wherein the pharmaceutical depot is formulated for controlled- and/or sustained-release of the PA over a period of 60 days.

10. The pharmaceutical depot according to claim 7, wherein the pharmaceutical depot is formulated for controlled- and/or sustained-release of the PA over a period of 90 days.

11. The pharmaceutical depot according to any one of claims 1 to 10, which is formulated for administration by injection, for example by intra-articular injection.

12. The pharmaceutical depot according to any one of claims 1 to 11, which is formulated for human medicine use.

13. The pharmaceutical depot according to any one of claims 1 to 11, which is formulated for veterinary use.

14. The pharmaceutical depot according to any one of claims 1 to 13, for the prevention or treatment of osteoarthritis.

15. Pharmaceutical depot according to any one of claims 1 to 13, for use in a method for the prevention or treatment of osteoarthritis.

## Patentansprüche

1. Pharmazeutisches Depot, umfassend (i) N-{5-[(Cyclopropylamino)carbonyl]-2-methylphenyl}-3-fluor-4-(pyridin-2-ylmethoxy)benzamid oder ein pharmazeutisch akzeptables Salz davon als ein pharmazeutisches Mittel (PM) und (ii) ein Polymer, das unter Erzeugung eines sauren Mikroklimas abgebaut wird, wobei das PM beim Polymerabbau von dem Polymer freigesetzt wird.

2. Pharmazeutisches Depot nach Anspruch 1, wobei das Polymer ausgewählt ist aus einem Polyester von einer Hydroxyfettsäure und Derivaten davon, einem Polymer von einem Alkyl-α-cyanoacrylat, einem Polyalkylenoxalat, einem Polyorthoester, einem Polycarbonat, einem Polyorthocarbonat, einer Polyaminsäure, einem Hyaluronsäureester und Mischungen davon.

3. Pharmazeutisches Depot nach Anspruch 2, wobei das Polymer ein Milchsäure-Glycolsäure-Copolymer ist.

4. Pharmazeutisches Depot nach Anspruch 3, wobei das Milchsäure-Glycolsäure-Copolymer ein Molverhältnis von Milchsäure : Glycolsäure im Bereich von 100:0 bis 50:50 aufweist.

5. Pharmazeutisches Depot nach Anspruch 4, wobei das Milchsäure-Glycolsäure-Copolymer ein Molverhältnis von Milchsäure : Glycolsäure von 95:5 aufweist.

6. Pharmazeutisches Depot nach Anspruch 4, wobei das Milchsäure-Glycolsäure-Copolymer ein Molverhältnis von Milchsäure : Glycolsäure von 50:50 aufweist.

7. Pharmazeutisches Depot nach irgendeinem der Ansprüche 1 bis 6, wobei das pharmazeutische Depot für die kontrollierte und/oder verzögerte Freisetzung des PM über einen Zeitraum von 30 bis 90 Tage formuliert ist.

8. Pharmazeutisches Depot nach Anspruch 7, wobei das pharmazeutische Depot für die kontrollierte und/oder verzögerte Freisetzung des PM über einen Zeitraum von 30 Tagen formuliert ist.

9. Pharmazeutisches Depot nach Anspruch 7, wobei das pharmazeutische Depot für die kontrollierte und/oder verzögerte Freisetzung des PM über einen Zeitraum von 60 Tagen formuliert ist.

10. Pharmazeutisches Depot nach Anspruch 7, wobei das pharmazeutische Depot für die kontrollierte und/oder verzögerte Freisetzung des PM über einen Zeitraum von 90 Tagen formuliert ist.

11. Pharmazeutisches Depot nach irgendeinem der Ansprüche 1 bis 10, das zur Verabreichung durch Injektion, z.B. durch intraartikuläre Injektion, formuliert ist.

12. Pharmazeutisches Depot nach irgendeinem der Ansprüche 1 bis 11, das für die humanmedizinische Verwendung formuliert ist.

13. Pharmazeutisches Depot nach irgendeinem der Ansprüche 1 bis 11, das für die veterinärmedizinische Verwendung formuliert ist.

14. Pharmazeutisches Depot nach irgendeinem der Ansprüche 1 bis 13 zur Vermeidung oder Behandlung von Osteoarthritis.

15. Pharmazeutisches Depot nach irgendeinem der Ansprüche 1 bis 13 zur Verwendung in einem Verfahren zur Vermeidung oder Behandlung von Osteoarthritis.

## Revendications

1. Produit pharmaceutique à effet retard comprenant (i) du *N*-{5-[(cyclopropylamino)carbonyl]-2-méthylphényl}-3-fluoro-4-(pyridin-2-ylméthoxy)-benz-amide, ou un sel pharmaceutiquement acceptable de celui-ci, en tant qu'agent pharmaceutique (AP) et (ii) un polymère qui se dégrade pour créer un microclimat acide, dans lequel l'AP est libéré du polymère après la dégradation du polymère.

2. Produit pharmaceutique à effet retard selon la revendication 1, où le polymère est choisi parmi un polyester d'acide gras hydroxylé et des dérivés de celui-ci, un polymère d'un α-cyanoacrylate d'alkyle, un oxalate de polyalkylène, un polyorthoester, un polycarbonate, un polyorthocarbonate, un polyacide aminé, un ester de l'acide hyaluronique, et des mélanges de ceux-ci.

3. Produit pharmaceutique à effet retard selon la revendication 2, où le polymère est un copolymère d'acide lactique-acide glycolique.

4. Produit pharmaceutique à effet retard selon la revendication 3, où le copolymère d'acide lactique-acide glycolique présente un rapport molaire d'acide lactique/acide glycolique dans la plage de 100:0 à 50:50.

5. Produit pharmaceutique à effet retard selon la revendication 4, où le copolymère d'acide lactique-acide glycolique présente un rapport molaire d'acide lactique/acide glycolique de 95:5.

6. Produit pharmaceutique à effet retard selon la revendication 4, où le copolymère d'acide lactique-acide glycolique présente un rapport molaire d'acide lactique/acide glycolique de 50:50.

7. Produit pharmaceutique à effet retard selon l'une quelconque des revendications 1 à 6, où le produit pharmaceutique à effet retard est formulé pour la libération contrôlée et/ou prolongée de l'AP sur une période de 30 à 90 jours.

8. Produit pharmaceutique à effet retard selon la revendication 7, où le produit pharmaceutique à effet retard est formulé pour la libération contrôlée et/ou prolongée de l'AP sur une période de 30 jours.

9. Produit pharmaceutique à effet retard selon la revendication 7, où le produit pharmaceutique à effet retard est formulé pour la libération contrôlée et/ou prolongée de l'AP sur une période de 60 jours.

10. Produit pharmaceutique à effet retard selon la revendication 7, où le produit pharmaceutique à effet retard est formulé pour la libération contrôlée et/ou prolongée de l'AP sur une période de 90 jours.

11. Produit pharmaceutique à effet retard selon l'une quelconque des revendications 1 à 10, qui est formulé pour une administration par injection, par exemple, par injection intra-articulaire.

12. Produit pharmaceutique à effet retard selon l'une quelconque des revendications 1 à 11, qui est formulé pour un usage en médecine humaine.

13. Produit pharmaceutique à effet retard selon l'une quelconque des revendications 1 à 11, qui est formulé pour un usage vétérinaire.

14. Produit pharmaceutique à effet retard selon l'une quelconque des revendications 1 à 13, pour la prévention ou le traitement de l'arthrose.

15. Produit pharmaceutique à effet retard selon l'une quelconque des revendications 1 à 13, pour son utilisation dans une méthode de prévention ou de traitement de l'arthrose.
